# EUROPEAN PATENT APPLICATION

(11) **EP 0 819 982 A1**
(43) Date of publication of application: **21.01.1998**
(21) Application number: 97304762.4
(22) Date of filing: 01.07.1997
(51) Int. Cl.: G03F 7/004, G03F 7/039

(54) **Radiation-sensitive composition**

(30) Priority: 18.07.1996 CH 1805/96
(71) Applicant: Olin Microelectronic Chemicals, Inc., Norwalk, Connecticut 06856-4500 (US)
(72) Inventor: Mertesdorf, Carl-Lorenz, Bad Krozingen (DE); Falcigno, Pasquale Alfred, 4052 Basle (CH)
(74) Representative: Bannerman, David Gardner

(57) **Abstract**

A radiation-sensitive composition containing as resin components a mixture of at least two phenolic resins, whose phenolic hydroxyl groups are partially replaced by specific acetal and/or ketal side groups, can be used as the positive resist formulation and is characterized by a good process stability as well as a high thermal stability in combination with a high flow stability.

## Description

### BACKGROUND OF THE INVENTION

### 1. Field of the Invention

The subject of the present invention is a radiation-sensitive composition containing a mixture of at least two partially protected phenolic resins and a photo-acid generator, a process for the production of positive images as well as the use of the radiation-sensitive composition as a positive resist for the production of printing plates, printed circuits, or integrated circuits.

### 2. Brief Description of Relevant Art

For the production of highly integrated circuits, it is presently necessary that structures with a width of less than 0.35 µm are able to be coated onto the substrate in an image-forming manner. The resolution capacity, for example, of an o-quinone diazide/novolak system has reached the limit of the physical possibilities for a wavelength of 365 mm, which is used for imaging exposure. For this reason, photoresists are increasingly of interest, which operate at shorter wavelengths in the deep UV region (DUV region, deep ultraviolet: 200 to 300 nm). Novolaks in this region absorb so intensely that a uniform exposure over a commonly used film thickness of approximately 1.0 µm is not possible. In order to assure the required optical transparency, poly(p-hydroxystyrene) or its derivatives are generally used as binder resins for photoresists operating in the DUV region.

Systems with increased radiation sensitivity, as is generally known, are comprised of, e.g., alkali-soluble binder resins, whose groups that mediate the alkali solubility, such as for example, hydroxyl groups, are blocked by acid-labile protective groups and thus make the binder resin extensively insoluble in alkali. Then by exposure in a primary reaction by means of a photo-acid generator, which absorbs at the appropriate wavelength, a strong acid forms, which leads to a cleavage of the protective groups in the subsequent secondary reaction and thus leads to a reformation of the groups that promote solubility.

As an example of such system, poly(p-hydroxystyrene) can be mentioned, whose phenol groups are protected with e.g. tert-butyloxycarbonyloxy (TBOC) or tetrahydropyranol groups (THP). Photoresists, which contain such binder resins are known (see, e.g.,: M.J. Bowden and S.R. Turner (Eds.) "Electronic and Photonic Application of Polymers", ACS Series 218, Washington 1988; as well as N. Hayashi et al., Polymer 33, 1583 (1992)), but have disadvantages relative to adhesion to silicon. Particularly with respect to the plasma-chemical structure transfer (etching process) onto the semiconductor substrate (TiN, SiO₂, Al, and the like) that follows in conjunction with the lithographic process, the loss of film thickness of these systems in the exposed regions of the resist film causes great problems (E. Reichmanis et al., J. Photopolym. Sci. Technol. 8, 709-728 (1995)).

In addition, these chemically reinforced positive resists are sensitive to the process interval between the primary reaction and the subsequent secondary reaction. Extended intervals (post exposure delay), which are already critical after a few minutes in sensitive systems, have a strong surface inhibition as a consequence, which leads to the formation of T-shaped denatured profiles (S.A. MacDonald et al., SPIE, Vol. 1466, Advances in Resist Technology and Processing VIII (1991) 2-7).

It has been attempted to improve adhesion, reproducibility and resolution by using a binder resin in resist formulations, whose phenolic hydroxyl groups have only partially been replaced by acid-cleavable protective groups. As an example, EP-A 447,868 will be mentioned, in which a radiation-sensitive composition is proposed, whose phenolic resin is partially protected by tetrahydropyranyl groups. In addition, a resin mixture of a phenolic resin and a novolak that is different from this are claimed as a binder.

In EP A 520,642, polyhydroxystyrene polymers partially protected by acetal or ketal groups are proposed, which will also be free of the above-named disadvantages. It is known, however, that in copolymers with free phenolic monomer units, increased thermal stability losses occur. The thermally induced decomposition of acid-labile protective groups (autocatalysis) is based on the catalytic effect of the weakly acidic phenolic hydroxyl groups. Acetal or ketal protective groups, however, are preferred, since they permit a longer interval between exposure and post exposure bake (also a greater period of play relative to the process) than the less acid-labile protective groups, such as, e.g., the TBOC group or the tert-butyl ester protective group.

The level of the glass-transition temperature (T_{g}) of the binder resin is of great importance for thermal flow stability. With increasing content of protective groups, the T_{g} decreases and consequently the flow stability of resist structures also decreases. A sufficiently high flow stability (form stability of the resist structures that are produced), however, is of basic importance for the etching process.

In EP-A 679,951, a resist is proposed, which contains as resin components a mixture, which is comprised of a polyhydroxystyrene partially protected with TBOC groups and a polyhydroxystyrene partially protected with acetal or ketal groups. The thermal stability of such resists, however, is insufficient.

The above-named problems cannot be satisfactorily resolved with the previously known resists. In particular, in the case of a relatively small content of protective groups, as is appropriate for high-resolving lithography, autocatalysis is problematical, and consequently the thermal stability of the resist is insufficient.

The task of the present invention is to develop new positive operating, highly active radiation-sensitive systems, particularly for the production of relief structures, which do not have the above-named disadvantages, i.e., they will possess above all a good process stability, they will be sensitive relative to UV, electron, and x-ray radiation, and they can be used particularly on the basis of their high optical transparency in the DUV region. In addition they will permit a high resolution and a small loss of layer thickness in the exposed regions as well as a good thermal stability in conjunction with a high flow stability.

### BRIEF SUMMARY OF THE INVENTION

It has now been found surprisingly that radiation-sensitive compositions, containing as resin components a mixture of at least two phenolic resins, whose phenolic hydroxyl groups are replaced partially by specific acetal and/or ketal side groups, do not have the above-described disadvantages.

The subject of the present invention is a radiation-sensitive composition containing the following, relative to the total quantity of components A, B and C,
(A) 9.9-95 wt.% of a phenolic resin, whose phenolic hydroxyl groups are replaced up to 10-90% by protective groups of formula I: 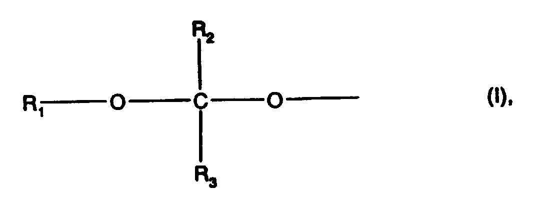 in which R₁ and R₂, independently of one another, indicate C₁-C₆ alkyl, C₃-C₆ cycloalkyl that is unsubstituted or substituted with one or more C₁-C₄ alkyl groups, or -(CH₂)ₙ aryl,
   R₃ indicates hydrogen, C₁-C₆ alkyl, C₃-C₆ cycloalkyl that is unsubstituted or substituted with one or more C₁-C₆ alkyl groups, or -(CH₂)ₙ aryl, or R₂ and R₃ together with the C-atom bound to this residue form a five to eight-member ring, which may contain, if necessary, other heteroatoms or groups, such as -O-, -S-, -SO₂- or -MR₈-, in which R₄ indicates hydrogen, C₁-C₆ alkyl, aryl, or C₆-C₁₆ aralkyl as ring members, whereby n is 0, 1, 2, or 3, and aryl stands for unsubstituted phenyl or naphthyl or these compounds substituted with one or more C₁-C₄ alkyl, C₁-C₄ alkoxy, nitro or cyano groups or halogen atoms;
(B) 4.9-90 wt.% of a phenolic resin, whose phenolic hydroxyl groups are replaced up to 10-90% by protective groups of formula II: 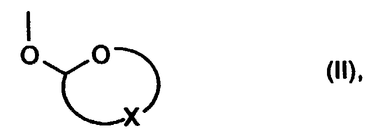 in which X stands for the following groups that are unsubstituted or substituted with one or more C₁-C₄ alkyl, C₁-C₄-alkoxy, nitro or cyano groups or halogen atoms: trimethylene, tetramethylene, pentamethylene, hexamethylene, 2-oxapropanediyl, 2-oxabutanediyl, 2-oxapentanediyl, 3-oxapentanediyl, 2-oxahexanediyl, 3-oxahexanediyl, 2-thiapropanediyl, 2-thiabutanediyl, 2-thiapentanediyl, 3-thiapentanediyl, 2-thiahexanediyl, 3-thiahexanediyl, 2-azapropanediyl, 2-azabutanediyl, 2-azapentanediyl, 3-azapentanediyl, 2-azahexanediyl, or 3-azahexanediyl groups; and
(C) 0.1-20.0 wt.% of a substance forming acid under the effect of actinic radiation.

### DETAILED DESCRIPTION OF PREFERRED EMBODIMENTS

A radiation-sensitive composition that contains the following is preferred:
(A) 9.9-95 wt.% of a phenolic resin containing 10-90 mol. % of repeating structural units of formula III and 10-90 mol.% of repeating structural units of formula IV: 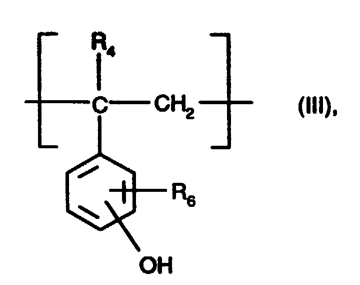 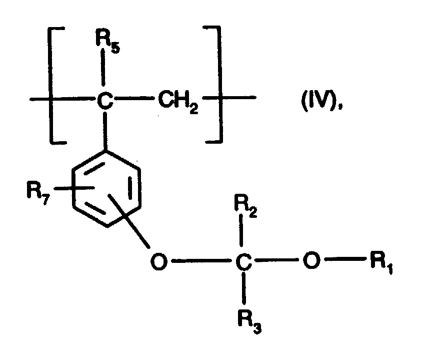 and
(B) 4.9-90 wt.% of a phenolic resin containing 10-90 mol.% of repeating structural units of formula III and 10-90 mol.% of repeating structural units of formula V: 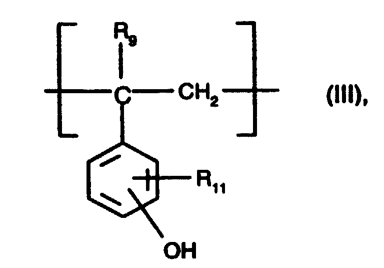 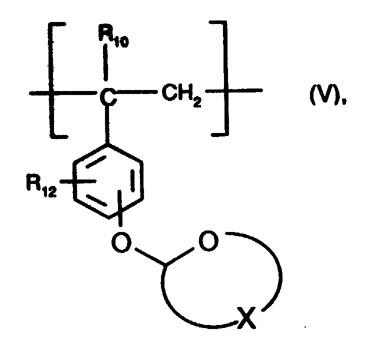
in which R₁, R₂, R₃ and X have the above-given meaning;
R₄, R₅, R₉ and R₁₀, independently of one another, represent hydrogen, methyl, or halogen; and
R₆, R₇, R₁₁ and R₁₂, independently of one another, stand for hydrogen, C₁-C₄ alkyl, C₁-C₄ alkoxy or halogen.

Particularly preferred is a radiation-sensitive composition containing:
(A) 9.9-95 wt.% of a phenolic resin containing 10-90 mol.% of repeating structural units of formula IIIa and 10-90 mol.% of repeating structural units of formula IVa: 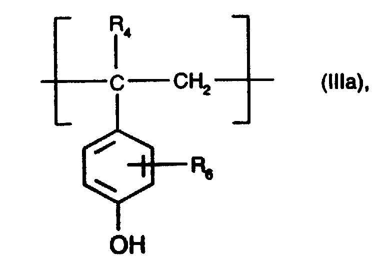 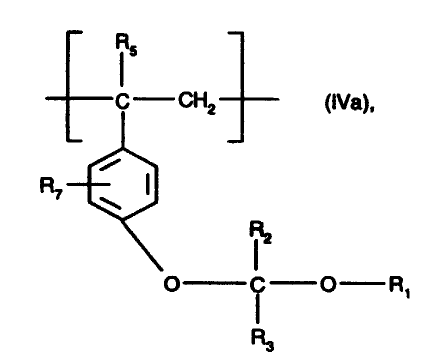 and
(B) 4.9-90 wt.% of a phenolic resin containing 10-90 mol.% of repeating structural units of formula IIIa and 10-90 mol.% of repeating structural units of formula Va: 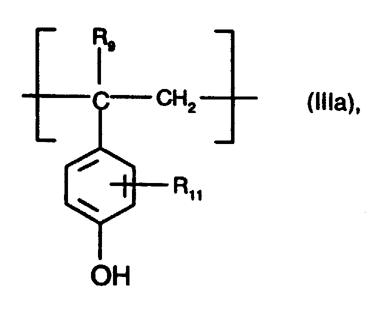 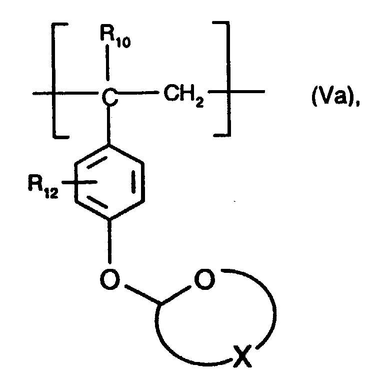
in which R₁ to R₇, R₉, to R₁₂ and X have the above-given meaning.

Alkyl groups as substituents R₁ to R₃, R₆, R₇, R₁₁ and R₁₂ may be linear or branched. The following can be named as examples: methyl, ethyl, n-propyl, isopropyl, n-butyl, sec-butyl, isobutyl, tert-butyl, n-pentyl, neo-pentyl, isopentyl, n-hexyl and isohexyl.

Alkoxy substituents, for example, are methoxy, ethoxy, n-propoxy, iso-propoxy, n-butoxy or tert-butoxy.

Halogen atoms as substituents R₄ to R₇ and R₉ to R₁₂, are preferably fluorine, chlorine and bromine, particularly chlorine.

Cycloalkyl groups are preferably cyclopentyl and cyclohexyl.

Examples of aryl groups are phenyl, tolyl, xylyl, mesityl, isityl, naphthyl and anthryl, whereby phenyl is particularly preferred.

Aralkyl groups are preferably benzyl or phenylethyl.

R₄ to R₇ and R₉ to R₁₂ in the structural units of formulas III, IV and V preferably stand for hydrogen.

In the protective groups of formula I, R₁ and R₂, independently of one another, preferably indicate methyl, ethyl, n-propyl, isopropyl, n-butyl, isobutyl, tert-butyl or cyclohexyl, and R₃ preferably stands for hydrogen or methyl.

In the protective groups of formula II, X preferably stands for trimethylene or tetramethylene.

The quantities of components (A), (B) and (C) to be used appropriately in the compositions of the invention may vary within wide ranges. Preferably, the composition of the invention contains the following, relative to the total quantity of components A, Band C: 39.8-92.0 wt.%, particularly 49.5-91.0 wt.% of component (A), 7.8-60.0 wt.%, particularly 8.5-50.0 wt.% of component (B), and 0.2-10.0 wt.%, particularly 0.5-8.0 wt.% of component (C).

In the radiation-sensitive compositions of the invention, the phenolic hydroxyl groups of the phenolic resin according to component (A) are replaced preferably up to 20 to 50% by protective groups of formula I and the phenolic hydroxyl groups of the phenolic resin according to component (B) are replaced preferably up to 10 to 40% by protective groups of formula II.

As components (A) or (B), phenolic resins are preferred, whose weight-average molecular weight lies between 3000 and 30,000 measured against polystyrene by means of gel permeation chromatography.

The phenolic resins according to component (A) or (B) may be obtained by known methods, for example, by subsequent protection of resins containing alkali-soluble phenolic groups with vinyl ether compounds or isopropenyl ether compounds, such as, e.g., with methyl vinyl ether, ethyl vinyl ether, n-butyl vinyl ether, t-butyl vinyl ether, cyclohexyl vinyl ether, isopropyl vinyl ether, 3,4-dihydro-2H-pyran, 2,3-dihydrofuran, 2-methoxypropene, and the like. The protection procedures are preferably conducted in the presence of a suitable, for the most part, acidic catalyst. Such suitable catalysts are, e.g., acidic ion exchanger resins, acids, such as, e.g., sulfonic acids, and hydrohalic acids or their salts, e.g., pyridinium tosylate or pyridinium chloride.

The following may be used as resins containing alkali-soluble phenolic groups: homopolymers, such as, e.g., poly(4-hydroxystyrene), poly(4-hydroxy-α-methylsytrene) and poly(3-hydroxystyrene), or copolymers of phenolic vinyl monomers and other monomer units, such as, e.g., polymers of 4-hydroxystyrene or 3-hydroxystyrene, with acrylic acid, methacrylic acid, alkylmethacrylate, alkylacrylate, styrene, fumaronitrile, maleic acid anhydride or maleinimide and their derivatives.

The alkali-soluble phenolic resins may be obtained according to known methods by suitable polymerization or copolymerization of monomers and may then be modified. Such modifications are, e.g., the partial hydrogenation of the phenolic groups to cyclohexanol groups or the partial substitution or derivatization of phenolic OH groups, e.g., by esterification or etherization.

As radiation-sensitive components (C) which form or cleave acids under the effect of actinic radiation, a large number of compounds are known. These include, for example, diazonium salts, such as are used in diazotype processes, o-quinone diazides, as they are used in known positive copier compounds, or also halogen compounds, which form hydrogen halide acids upon irradiation. Compounds of this type are described, for example, in US-A 3,515,552; 3,536,489; or 3,779,778, as well as in DB-A 2,718,259; 2,243,621; or 2,610,842.

As radiation-sensitive components (C), of the composition of the invention, cationic photoinitiators from the group of onium salts, particularly iodonium or sulfonium salts, are particularly suitable. Such compounds are described, for example, in "UV-Curing, Science, and Technology" (Editor: S.P. Pappas, Technology Marketing Corp., 642 Westover Rd., Stanford, Connecticut, USA).

Further, sulfoxonium salts can be used as radiation-sensitive compounds. Such salts are described, for example, in EP-B 35,969 or in EP-A 44,274; or 54,509. Particularly to be mentioned are aliphatic sulfoxonium salts, which absorb in the deep UV region.

Further, sulfonic acid esters, such as those described, for example, in US 5,118,582; US 5,189,402, and in T. Ueno et al., Polym. Eng. Sci. 32, 1511 (1992) may also be used. Further, other sulfonic acid esters are suitable, such as, for example, N-sulfonyloxyimide, as described in EP-A 502,677, and nitrobenzylsulfonates, as described in US 5,135,838. Other applicable sulfonyl compounds are given, for example, in DE-A 4,225,422 and in Polym. Eng. Sci. 32, 1476 (1992).

The following are particularly preferred for irradiation with short-wave UV beams or electron beams: disulfone compounds, such as, e.g., phenylcumyldisulfone and phenyl-(4-anisyl)disulfone, as described in DE 3,804,316.

Further, iminosulfates are particularly suitable, such as described, e.g., in EP-A 241,423 and BP-A 571,330.

Compounds, which release sulfonic acid upon irradiation with actinic light, can also be used. Such compounds are known in and of themselves and are described, for example, in GB-A 2,120,263, in BP-A 84,515; 37,152; or 58,638 as well as in US-A 4,258,121; or 4,371,605. Compounds, which release carboxylic acid upon irradiation, may also be used. Such compounds are described, e.g., in EP-A 552,548.

If salts are used as radiation-sensitive acid-cleaving components (C), then these are preferably soluble in organic solvents. Particularly preferred for these salts are products of complex acids, for example, hydrofluoboric acid, hexafluorophosphoric acid, trifluoromethanesulfonic acid, hexafluoroarsenic acid or hexafluoro-antimony acid.

Particularly preferred as components (C) are iodonium or sulfonium salts.

In addition to components (A), (B) and (C), the radiation-sensitive compositions according to the invention preferably also contain an organic solvent as component (D).

The selection of organic solvent according to component (D) and its concentration are directed primarily according to the type of composition of the resist formulation and according to the coating process. The solvent will be inert, i.e., it will enter into no chemical reaction with components (A) (B) and (C), and it will be able to be removed again in the drying after the coating. Suitable solvents are, e.g., ketones, ethers, esters, and aromatic compounds, as well as any mixtures thereof.

Examples of these are methyl ethyl ketone, isobutyl methyl ketone, cyclopentanone, cyclohexanone, N-methylpyrrolidone, dioxane, tetrahydrofuran, 2-ethoxyethanol, 2-ethoxyethanol; acetates, such as butyl acetate; 1-methoxy-2-propanol, 1,2-dimethoxyethane, diethylene glycol dimethyl ether, butylglycol, alkylene glycol monoalkyl ethers, such as, e.g., ethyl cellosolve, ethylene glycol monobutyl ether and methyl cellosolve; alkylene glycol alkyl ether esters, such as methyl cellosolve acetate, ethyl cellosolve acetate, propylene glycol ethyl ether acetate, and methoxypropyl acetate; acetic acid ethyl ester, acetic acid n-butyl ester, 3-ethoxypropionic acid ethyl ester, as well as methoxymethyl propionate, ethyl lactate, toluene and xylenes.

Preferred organic solvents are ethyl lactate, ethoxyethyl propionate, and particularly methoxypropyl acetate.

Also preferred are compositions containing an additional 0.01 to 40 wt.% of the usual additives as component (E), relative to the total amount of components (A), (B), and (C).

The usual additives include, for example, the following substances:
Pigments or colorants in quantities of approximately 0.1 to 2 wt.%, relative to the total amount of components (A), (B) and (C), as well as Microlith Blue 4G, Orasol Blue GN And Irgalith Green;
Inorganic or organic fillers in quantities of approximately 5 to 15 wt.% relative to the total quantity of components (A), (B) and (C), such as talcum, quartz (SiO₂), barium sulfate (BaSO₄), aluminum oxide and calcium carbonate, with which, e.g., the properties of a coating, such as heat stability, adhesion or scratch capacity, can be improved.

Weak basic additives (resist additives) in a total quantity of approximately 0.01 to 10 wt.% relative to the total quantity of components (A), (B) and (C), such as anti-foaming agents (e.g., Byk 80), adhesion mediators (e.g., benzotriazole), fungicides and thixotropic agents or hydroxyterminated polyglycol ethers with ethylene oxide and/or propylene oxide units, such as, for example, Tetronic 701, 901, 908 P and 1501 (products of BASF);
Surface-active agents for improving the wetting capacity of the compound, for preventing streaking formation on the formed film, for improving the developability of the irradiated region, etc. The surface-active agents comprise nonionic surfactants, such as, for example, polyoxyethylene lauryl ether, polyoxyethylene stearyl ether, polyoxyethylene oleyl ether, polyoxyethyleneoctyl phenol ether, polyoxyethylenenonylphenyl ether, polyoxyethylene glycol dilaurate, polyethyleneglycol distearate; fluorine-containing surfactants, such as, for example, F Top EF 301, EF 303, and EF 352 (products of Shin Akita Kasei K.K.), Megafac F 171 and F 173 (products of Dainippon Ink & Chemicals), Fluorad FC 430 and FC 431 (products of Sumitomo 3M Limited), Asahi Guard AG 710, Surflon S-382, Surflon SC 101, SC 102, SC 103, SC 104, SC 105 and SC 106 (products of Asahi Glass Co., Ltd). In addition, for example, the organosiloxane polymer KP 341 (product of Shin-Etsu Chemical Co., Ltd.) and Polyflow No. 75 and No. 95 (products of Kyoeisha Yusikagaku Kogyo K.K.) are used, Which are acrylic or methacrylic acid polymers. The quantity of surface-active agent used amounts to approximately 0.01 to 0.1 wt.% relative to the total quantity of components (A) and (B);
Strongly basic additives, such as aromatic or aliphatic amines, ammonium salts, or N-containing heterocyclic compounds, usually in a concentration of 0.01 to 1 wt.% relative to the total quantity of components (A), (B), and (C);

The production of the resist formulations according to the invention are produced, e.g., by mixing components (A), (B) (C) and, if necessary, (D) and (E), while stirring at room temperature, whereby a homogeneous solution is obtained.

As indicated initially, the compositions according to the invention find application as positive resist formulations for electronics (galvanoresist, etching resist, solder top resist), for application in preform etching, for the production of printing plates, such as offset printing plates or screen printing plates, and printed circuits, and particularly in microelectronics for the production of integrated circuits.

The mixtures according to the invention are particularly characterized by a high thermal stability and possess a good storage stability as well as a high resolution capacity and a good adhesion.

Further, it is particularly preferred that the resin mixtures of components (A) and (B) have high glass transition temperatures and high decomposition temperatures.

The resist formulation is introduced by means of known coating processes at room temperature uniformly onto the substrate, e.g., by dipping, doctor-blade coating, brushing on, spraying, particularly by electrostatic spraying and reverse-roll coating and most preferably by spinning.

The coating amount (layer thickness) and the type of substrate (film carrier) are dependent on the desired field of application. The range of film thickness generally comprises values of 0.1 up to more than 10 mm, preferably 0.2 to 2.0 mm.

In microelectronics, the substrate is, for example, a silicon wafer that is oxidized on the surface.

After the coating, the solvent is generally removed by drying, e.g., at temperatures between 70 and 130°C.

The resist film represents a photosensitive resist, which has a high sensitivity to actinic radiation after drying and has a very good adherence to the substrate. Further, it has a high transparency and sensitivity even in the deep UV region, particularly at 250 nm, and has a good thermal stability.

In order to produce relief structures, the substrate coated with the composition according to the invention is exposed to light in an image-forming manner. The term "image-forming" exposure includes both the exposure through a photomask, which contains a prespecified pattern, for example, a slide, as well as exposure with a laser beam, which is, for example, moved in a computer-controlled manner over the surface of the coated substrate. In this way, an image is produced by irradiation with the computer-controlled electron beam as well as exposure with x-ray or UV radiation through an appropriate mask.

As a rule, UV and/or VIS irradiation is used for exposure, particularly of wavelengths between approximately 190 and 1,000 nm, particularly 190 to 300 nm and most particularly at 250 nm. For irradiation, in principle, all radiation sources known in and of themselves may be utilized, for example, mercury high-pressure lamps or UV/VIS lasers and particularly excimer lasers (KrF excimer laser light of wavelength 248 nm). As a radiation source, x-ray beams (for example, synchrotron beams) or radiation of charged particles (for example, electron beams), among others, may also be used. The process parameters, such as, e.g., irradiation time and distance between the radiation source and the radiation-sensitive layer, are generally dependent on the type of radiation-sensitive composition and on the desired properties of the coating, and may be established by the expert with a few routine tests.

After image-forming exposure, the wafer is heated, if necessary, at 50 to 150°C for a few seconds up to several minutes (post exposure bake).

Subsequently, the exposed places of the photoresist are removed by dissolving out in a developer. The selection of the respective developer is guided according to the type of photoresist, particularly according to the nature of the binder agent used or the photolysis products that form. The developer may comprise aqueous solutions of bases, to which, if necessary, wetting agents and organic solvents or their mixtures are added.

The compositions of the invention are preferably used as positive resists. Thus, a process for the production of positive images forms another subject of the invention, which contains the following process measures:
(I.) Coating of a substrate with a radiation-sensitive composition of the invention containing components (A), (B), and (C) ;
(II.) Exposure of the coated substrate to actinic radiation in a predetermined pattern; and
(III.) Development of the irradiated substrate with a developer for positive resists.

Particularly preferred as developers are aqueous alkaline solutions, such as are used also for the development of o-quinone diazide/novolak resist coatings. These include, e.g., aqueous solutions of alkali metal silicates, phosphates, hydroxides and carbonates, but particularly tetraalkylammonium hydroxide solutions, such as, e.g., tetramethylammonium hydroxide solution.

If necessary, small quantities of wetting agents and/or organic solvents may be added to these solutions. Typical inorganic solvents, which can be added to the developer fluids, are, for example, cyclohexanone, 2-ethoxyethanol, acetone, isopropanol, ethanol, as well as mixtures of two or more of these solvents.

The application of the developer is preferably conducted by one-time dipping of the substrate that has been coated and exposed in an image-forming manner into the developer solution, by spraying the developer solution or by repeated introduction and spinning of the developer onto the substrate that has been coated and exposed in an image-forming manner.

The subject of the invention is also the use of the compositions of the invention as positive resists for the production of printing plates, printed circuits or integrated circuits, as well as the printing plates, printed circuits, or integrated circuits produced with the use of the compositions of the invention.

### EXAMPLES

The following Examples further illustrate the present invention. All parts and percentages are by weight and all temperatures are degrees Celsius unless explicitly stated otherwise.

The following abbreviations are used in the examples:
- TGA:: thermogravimetric analysis
- DSC:: differential scanning calorimetry

### I. Synthesis of phenolic resins:

### Synthesis Example 1:

### Preparation of poly(4-(1-ethoxyethoxy)styrene/4-hydroxystyrene) with a protective group content of 37.5 mol.%.

60.0 g of poly-4-hydroxystyrene (M_{w} = 8300) are dissolved in 247.0 g of tetrahydrofuran and stirred together with 16.0 g of ethyl vinyl ether and a catalytic quantity of p-toluene-4-sulfonic acid for 16 hours at room temperature. Then the catalyst is removed from the reaction solution with a basic ion exchanger. The polymer is precipitated out by casting the reaction solution into water and is filtered off, washed with water, and then dried to constant weight. 72.0 g of poly(4-(1-ethoxyethoxy)styrene/4-hydroxystyrene) are obtained, in which 37.5% of the OH groups are protected, according to TGA analysis.

Decomposition temperature (onset): 241°C (TGA, heating rate of 10°C/min)
Glass-transition temperature: 105°C (DSC, heating rate of 10°C/min).

### Synthesis Example 2:

### Preparation of poly (4-(1-ethoxyethoxy)styrene/4-hydroxystyrene) with a protective group content of 35.0 mol.%.

Analogously to Synthesis Example 1, 71.2 g of poly(4-(1-ethoxyethoxy) styrene/4-hydroxystyrene) are prepared from 60.0 g of poly-4-hydroxystyrene (M_{w} = 8300) and 14.9 g of ethyl vinyl ether, and 35.0% of the OH groups are protected, according to TGA analysis.

Decomposition temperature (onset): 236°C (TGA, heating rate of 10°C/min)
Glass-transition temperature: 109°C (DSC, heating rate of 10°C/min).

### Synthesis Example 3:

### Preparation of poly(4-(2-tetrahydropyranyloxy)styrene/4-hydroxystyrene) with a protective group content of 31.0 mol.%.

60.0 g of poly-4-hydroxystyrene (M_{w} = 8300) are dissolved in 247.0 g of tetrahydrofuran and stirred together with 18.7 g of 3,4-dihydro-2H-pyran and a catalytic quantity of p-toluene-4-sulfonic acid for 16 hours at room temperature. Then the catalyst is removed from the reaction solution with a basic ion exchanger. The polymer is precipitated out by casting the reaction solution into water and is filtered off, washed with water, and then dried to constant weight. 71.6 g of poly(4-(2-tetrahydropyranyloxy)styrene/4-hydroxystyrene) are obtained, in which 31.0% of the OH groups are protected, according to TGA analysis.

Decomposition temperature (onset): 174°C (TGA, heating rate of 10°C/min)
Glass-transition temperature: cannot be measured (>140°C).

### Synthesis Example 4:

### Preparation of poly(4-(2-tetrahydropyranyloxy)styrene/4-hydroxystyrene) with a protective group content of 25.0 mol.%.

Analogously to Synthesis Example 3, 69.0 g of poly(4-(2-tetrahydropyranyloxy)styrene/4-hydroxystyrene) are prepared from 60.0 g of poly-4-hydroxystyrene (M_{w} = 8300) and 15.4 g of 3,4-dihydro-2H-pyran, and 25.0% of the OH groups are protected, according to TGA analysis.

Decomposition temperature (onset): l73°C (TGA, heating rate of 10°C/min)
Glass-transition temperature: cannot be measured (>140°C).

### Synthesis Example 5:

### Preparation of poly(4-(2-tetrahydropyranyloxy)styrene/4-hydroxystyrene) with a protective group content of 17.5 mol.%.

Analogously to Synthesis Example 3, 66.2 g of poly(4-(2-tetrahydropyranyloxy)styrene/4-hydroxystyrene) are prepared from 60.0 g of poly-4-hydroxystyrene (M_{w} = 8300) and 10.8 g of 3,4-dihydro-2H-pyran, and 17.5% of the OH groups are protected, according to TGA analysis.

Decomposition temperature (onset): 159°C (TGA, heating rate of 10°C/min)
Glass-transition temperature: cannot be measured (>140°C).

### Example of resin mixtures:

### Resin Mixtures 1:

2.0 g of a resin mixture consisting of the polymer from Synthesis Example 2 (resin (a)) and the polymer from Synthesis Example 3 (resin (b)) each time are dissolved in 8.0 g of tetrahydrofuran. The resin mixtures are precipitated by casting the respective solutions into water, filtering off, washing with water, and then drying to constant weight. The respective mixing ratios are compiled in Table 1 together with the decomposition temperatures T_{dec} (TGA, heating rate of 10°C/min) and the glass-transition temperatures T_{g} (DSC, heating rate of 10°C/min).

**Table 1**

| Mixture | Resin (a) [g] | Resin (b) [g] | T_{dec} (onset) [°C] | T_{g} [°C] |
|---|---|---|---|---|
| 1 | 1.6 | 0.4 | 232 | 115 |
| 2 | 1.4 | 0.6 | 231 | 119 |
| 3 | 1.2 | 0.8 | 230 | 121 |
| 4 | 1.0 | 1.0 | 229 | 122 |

### Resin Mixtures 2:

Analogously to Resin Mixtures 1, other resin mixtures consisting of the polymer from Synthesis Example 1 (resin (a)) and the polymer from Synthesis Example 4 (resin (b)) are prepared in various mixing ratios, which are compiled in Table 2, together with the decomposition temperatures T_{dec} (TGA, heating rate of 10°C/min) and the glass-transition temperatures T_{g} (DSC, heating rate of 10°C/min).

**Table 2**

| Mixture | Resin (a) [g] | Resin (b) [g] | T_{dec} (onset) [°C] | T_{g} [°C] |
|---|---|---|---|---|
| 1 | 1.6 | 0.4 | 235 | 114 |
| 2 | 1.4 | 0.6 | 232 | 117 |
| 3 | 1.2 | 0.8 | 232 | 120 |

### Resin Mixtures 3:

Analogously to Resin Mixtures 1, other resin mixtures consisting of the polymer from Synthesis Example 1 (resin (a)) and the polymer from Synthesis Example 5 (resin (b)) are prepared in various mixing ratios, which are compiled in Table 3 together with the decomposition temperatures T_{dec} (TGA, heating rate of 10°C/min) and the glass-transition temperatures T_{g} (DSC, heating rate of 10°C/min).

**Table 3**

| Mixture | Resin (a) [g] | Resin (b) [g] | T_{dec} (onset) [°C] | T_{g} [°C] |
|---|---|---|---|---|
| 1 | 1.8 | 0.2 | 235 | 109 |
| 2 | 1.7 | 0.3 | 234 | 111 |
| 3 | 1.6 | 0.4 | 232 | 113 |
| 4 | 1.4 | 0.6 | 232 | 116 |

### Application Example 1:

98.80 g of a resin mixture (mixture 1 according to Table 3), 1.0 g of triphenylsulfonium trifluoromethanesulfonate and 0.20 g of triphenylimidazole are dissolved in 456 g of 1-methoxy-2-propylacetate (resist solution 1). The solution is filtered through a filter with a pore diameter of 0.20 µm and is spun onto a silicon wafer, in such a way that a film with a film thickness of 0.76 µm is obtained after 60 seconds of preliminary drying at 130°C on the hot plate. Exposure is conducted with a 4:1 projection illuminator (ISI XLS 7800/3/53, NA 0.53) with KrF excimer laser radiation (248 nm) through a chromium quartz mask in steps of 3 mJ/cm³. Then the wafer is heated for 90 seconds long at 110° on the hot plate and then developed for 60 seconds in commercially available 0.262 N tetramethylammonium hydroxide solution. With an exposure dosage of 34 mJ/cm², image-true, perpendicular 0.22 µm l/s structures are obtained with resist 1. The thermal flow stability of resist 1, i.e., the temperature at which the profile with a width of 20 µm does not notably deform, amounts to 120°C. In order to determine the decomposition temperature T_{dec} of resist 1, a resist film, as described above, is produced on a silicon wafer. After the preliminary drying, the resist film is mechanically removed from the wafer and measured by means of TGA (heating rate of 10°C/min). The decomposition temperature (onset) amounts to 230°C.

### Application Example 2:

98.80 g of a resin mixture (mixture 3 according to Table 3), 1.0 g of triphenylsulfonium trifluoromethanesulfonate and 0.20 g of triphenylimidazole are dissolved in 456 g of 1-methoxy-2-propylacetate (resist solution 2). The solution is filtered through a filter with a pore diameter of 0.20 µm and processed onto a silicon wafer, as described in Application Example 1. With an exposure dose of 30 mJ/cm2, image-true, perpendicular 0.22 µm l/s structures are obtained with resist 2. The thermal flow stability of resist 2, i.e., the temperature at which the profile with a width of at least 2 µm does not notably deform, amounts to 125°C. In order to determine the decomposition temperature T_{dec} of resist 2, a resist film, such as described above, is produced on a silicon wafer. After preliminary drying, the resist film is mechanically removed from the wafer and measured by means of TGA (heating rate of 10°C/min). The decomposition temperature (onset) amounts to 230°C.

### Application Example 3 (comparative):

98.80 g of the copolymer prepared in synthesis Example 1, 1.0 g of triphenylsulfonium trifluoromethanesulfonate and 0.20 g of triphenylimidazole are dissolved in 456 g of 1-methoxy-2-propylacetate (resist solution 3). The solution is filtered through a filter with a pore diameter of 0.2 µm and processed onto a silicon wafer, as described under application example 1. The process is conducted analogously to the conditions named in application example 1. For an exposure dose of 30 mJ/cm², image-true, perpendicular 0.22 µm l/s structures are obtained with resist 3. The thermal flow stability of resist 3 amounts to only 110°C. The decomposition temperature (onset) amounts to 230°C.

### Application Example 4 (comparative):

98.80 g of the copolymer prepared in Synthesis Example 4, 1.0 g of triphenylsulfonium trifluoromethanesulfonate, and 0.20 g of triphenylimidazole are dissolved in 456 g of 1-methoxy-2-propylacetate (resist solution 4) . The solution is filtered through a filter with a pore diameter of 0.2 µm and processed onto a silicon wafer, as described under application example 1. The process is conducted analogously to the conditions named in application example 1. For an exposure dose of 76 mJ/cm², structures with a resolution of only 0.30 µm l/s with oblique profiles are obtained with resist 4. The thermal flow stability of resist 4 amounts to more than 150°C. The decomposition temperature (onset) amounts to only 165°C.

### Application Example 5:

94.6 g of a resin mixture (mixture 2, according to Table 3), 5.0 g of [2-nitro-6(trifluoromethyl)benzyl]-4-methoxybenzenesulfonate and 0.40 g of triphenylimadazole are dissolved in 456 g of 1-methoxy-2-propylacetate (resist solution 5). The solution is filtered through a filter with a pore diameter of 0.2 µm and processed onto a silicon wafer, as described under application Example 1. For an exposure dose of 50 mJ/cm², image-true, perpendicular 0.22 µm l/s structures are obtained with resist 5. The thermal flow stability of resist 5 amounts to 125°C. The decomposition temperature (onset) amounts to 230°C.

All patent applications, patents, and other publications cited herein are incorporated by reference in their entirety. While the invention has been described above with reference to specific embodiments thereof, it is apparent that many changes, modifications, and variations can be made without departing from the inventive concept disclosed herein. Accordingly, it is intended to embrace all such changes, modifications, and variations that fall within the spirit and broad scope of the appended claims.

## Claims

1. Composition containing the following, relative to the total quantity of components A, B and C,
(A) 9.9-95 wt.% of a phenolic resin, whose phenolic hydroxyl groups are replaced up to 10-90% by protective groups of formula I: 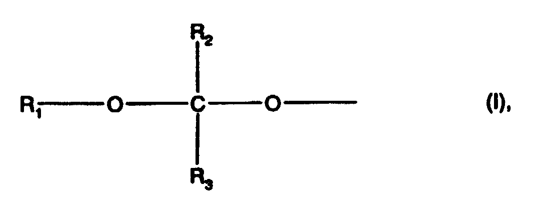 in which R₁ and R₂, independently of one another, indicate C₁-C₆ alkyl, C₃-C₆ cycloalkyl that is unsubstituted or substituted with one or more C₁-C₄ alkyl groups, or -(CH₂)ₙ aryl,
R₃ indicates hydrogen, C₁-C₆ alkyl, C₃-C₆ cycloalkyl that is unsubstituted or substituted with one or more C₁-C₄ alkyl groups, or -(CH₂)ₙ aryl, or R₂ and R₃ together with the C-atom bound to this residue form a five to eight-member ring, which may contain, if necessary, other heteroatoms or groups, such as -O-, -S-, -SO₂- or -NR₈-, in which R₈ indicates hydrogen, C₁-C₆ alkyl, aryl, or C₆-C₁₆ aralkyl as ring members, whereby n is 0, 1, 2, or 3, and aryl stands for unsubstituted phenyl or naphthyl or these compounds substituted with one or more C₁-C₄ alkyl, C₁-C₄ alkoxy, nitro or cyano groups or halogen atoms;
(B) 4.9-90 wt.% of a phenolic resin, whose phenolic hydroxyl groups are replaced up to 10-90% by protective groups of formula II: 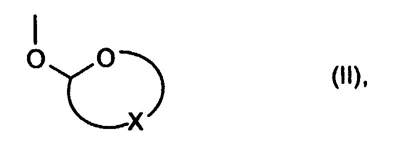 in which X stands for the following groups that are unsubstituted or substituted with one or more C₁-C₄ alkyl, C₁-C₄-alkoxy, nitro or cyano groups or halogen atoms: trimethylene, tetramethylene, pentamethylene, hexamethylene, 2-oxapropanediyl, 2-oxabutanediyl, 2-oxapentanediyl, 3-oxapentanediyl, 2-oxahexanediyl, 3-oxahexanediyl, 2-thiapropanediyl, 2-thiabutanediyl, 2-thiapentanediyl, 3-thiapentanediyl, 2-thiahexanediyl, 3-thiahexanediyl, 2-azapropanediyl, 2-azabutanediyl, 2-azapentanediyl, 3-azapentanediyl, 2-azahexanediyl, or 3-azahezanediyl groups; and
(C) 0.1-20.0 wt.% of a substance forming acid under the effect of actinic radiation.

2. Composition according to Claim 1, containing
(A) 9.9-95 wt.% of a phenolic resin containing 10-90 mol.% of repeating structural units of formula III and 10-90 mol.% of repeating structural units of formula IV: 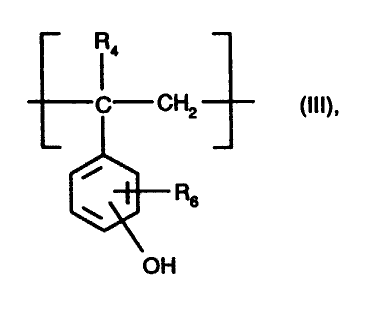 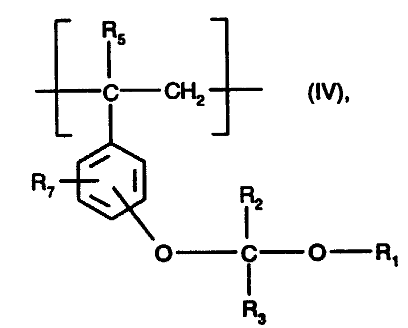 and
(B) 4.9-90 wt.% of a phenolic resin containing 10-90 mol.% of repeating structural units of formula III and 10-90 mol.% of repeating structural units of formula V: 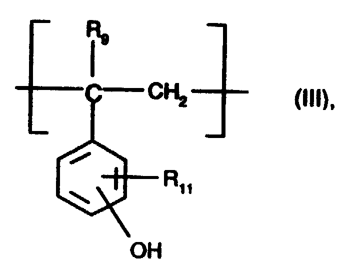 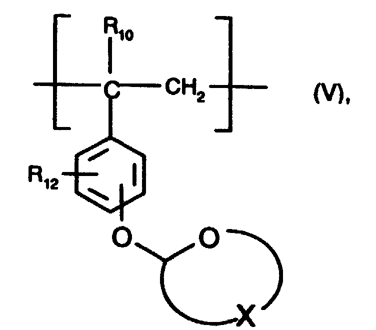
in which R₁, R₂, R₃ and X have the meaning indicated in Claim 1,
R₄, R₅, R₉ and R₁₀, independently of one another, represent hydrogen, methyl, or halogen; and
R₆, R₇, R₁₁ and R₁₂ independently of one another, stand for hydrogen, C₁-C₄ alkyl, C₁-C₄ alkoxy or halogen.

3. Composition according to Claim 2 containing
(A) 9.9-95 wt.% of a phenolic resin containing 10-90 mol.% of repeating structural units of formula IIIa and 10-90 mol.% of repeating structural units of formula IVa: 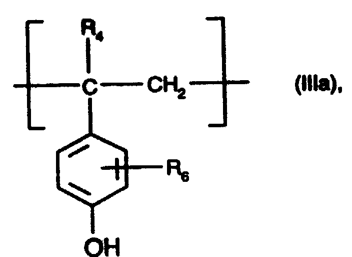 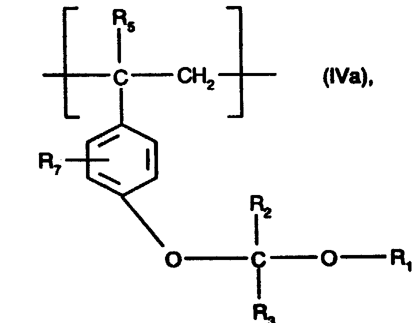 and
(B) 4.9-90 wt.% of a phenolic resin containing 10-90 mol.% of repeating structural units of formula IIIa and 10-90 mol.% of repeating structural units of formula Va: 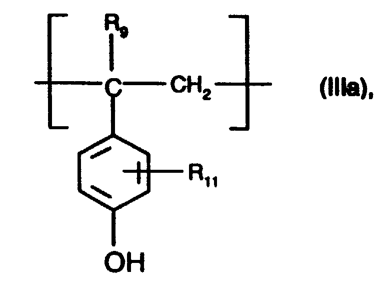 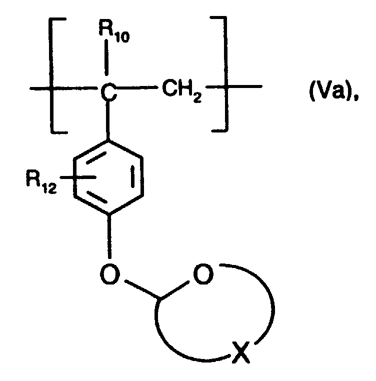
in which R₁ to R₇, R₉ to R₁₂ and X have the meaning given in Claim 2. 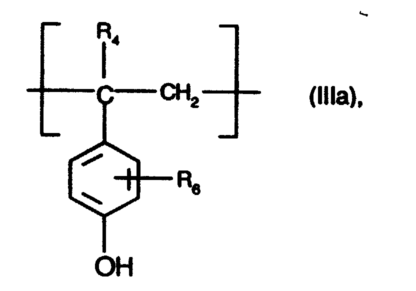 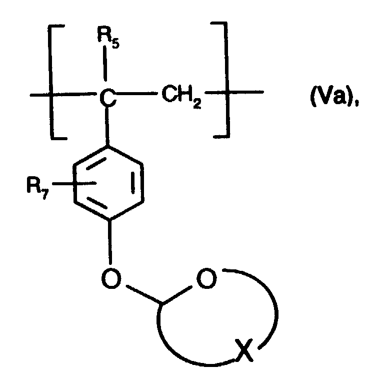 in which R₁ to R₇ and X have the meaning given in Claim 2.

4. Composition according to Claim 2 containing
(A) 9.9-95 wt.% of a phenolic resin containing 10-90 mol.% of repeating structural units of formula III and 10-90 mol.% of repeating structural units of formula IV and
(B) 4.9-90 wt.% of a phenolic resin containing 10-90 mol.% of repeating structural units of formula III and 10-90 mol.% of repeating structural units of formula V,
in which R₄, R₅, R₆ and R₇ indicate hydrogen.

5. Composition according to Claim 1 containing
(A) 9.9-95 wt.% of a phenolic resin, whose phenolic hydroxyl groups are replaced up to 10-90% by protective groups of formula I, in which R₁ and R₂, independently of one another, indicate methyl, ethyl, n-propyl, isopropyl, n-butyl, isobutyl, tert-butyl or cyclohexyl, and R₃ stands for hydrogen or methyl, and
(B) 4.9-90 wt.% of a phenolic resin, whose phenolic hydroxyl groups are replaced up to 10-90% by protective groups of formula II, in which X stands for trimethylene or tetramethylene.

6. Composition according to Claim 1 containing 39.8-92.0 wt.% of component (A), 7.8-60.0 wt.% of component (B), and 0.2-10.0 wt.% of component (C).

7. Composition according to Claim 1 containing an iodonium or sulfonium salt as component (C).

8. Process for the production of positive images by
(I.) Coating a substrate with a radiation-sensitive composition of the invention according to Claim 1,
(II.) Exposing the coated substrate to actinic radiation in a predetermined pattern; and
(III.) Developing the exposed substrate with a developer for positive resists.

9. The printing plates, printed circuits or integrated circuits produced with the use of the radiation-sensitive composition according to Claim 1.
